# EUROPÄISCHE PATENTSCHRIFT

(11) **EP 2 034 933 B1**
(45) Veröffentlichungstag und Bekanntmachung des Hinweises auf die Patenterteilung: **21.12.2011**
(21) Anmeldenummer: 07764766.7
(22) Anmeldetag: 21.06.2007
(51) Int. Cl.: A61F 9/00, C08F 120/06, G02B 1/04

(54) **OPHTHALMOLOGISCHE ZUSAMMENSETZUNG UND IHRE VERWENDUNG**
OPHTHALMOLOGICAL COMPOSITION AND USE THEREOF
COMPOSITION OPHTALMOLOGIQUE ET SON UTILISATION

(30) Priorität: 21.06.2006 DE 102006028507
(43) Veröffentlichungstag der Anmeldung: 18.03.2009
(73) Patentinhaber: Carl Zeiss Meditec AG, 07745 Jena (DE)
(72) Erfinder: NACHBAUR, Jürgen, 13509 Berlin (DE); NACHBAUR, Lidia, 13509 Berlin (DE); HUTTENLOCHNER, Markus, 87766 Memmingerberg (DE)
(74) Vertreter: Lechner, Armin Anton
(86) Internationale Anmeldenummer: PCT/EP2007/005479
(87) Internationale Veröffentlichungsnummer: WO 2007/147599

(56) Entgegenhaltungen:
- EP-A- 0 431 868
- WO-A-97/35896
- CA-A1- 2 404 522
- US-A1- 2006 122 349

## Beschreibung

Die Erfindung betrifft eine ophthalmologische Zusammensetzung sowie ihre Verwendung, insbesondere als Augenimplantat, wie Intraokularlinse.

Aus der US 2006/0122349 A1 ist eine ophthalmologische Zusammensetzung bekannt, welche ein hochbrechendes Monomer mit einer Carbazol- oder Naphtyl-Gruppe, ein hydrophiles Monomer, einen Vernetzer, einen UV-Absorber sowie einen Farbstoff umfasst.

Es ist bekannt, die Netzhaut des Auges gegen phototoxische Einflüsse von Strahlung im Ultraviolettbereich und violettem Lichtbereich mittels Absorber zu schützen. Derartige Absorber können im optischen Bereich von Intraokularlinsen vorgesehen sein. Auf dem Markt befindliche Intraokularlinsen absorbieren insbesondere im violettem Lichtbereich nur teilweise, sodass größenordnungsmäßig 25 % bis 35 % des Lichtes mit 430 nm durch das Linsenmaterial hindurchtreten.

Untersuchungen zeigen, dass der violette Lichtanteil eine entscheidende Rolle beim Absterben von RPE (retinales Pigmentepithel)-Zellen durch Apoptosis spielt. Im Laufe der Zeit kann dies zu altersbedingter Makuladegeneration fuhren.

Andererseits ist für das Sehen bei verminderten Lichtbedingungen (Scotopic vision), d.h. beim Dämmerungssehen und Nachtsehen, die Durchlässigkeit im blauem Lichtspektrum (etwa 450 nm bis 500 nm) bedeutsam. In diesem blauen Wellenlängenbereich soll so wenig wie möglich Licht beim Dämmerungssehen und Nachtsehen absorbiert werden. Bekannte auf dem Markt befindliche Intraokularlinsen haben in diesem Bereich (z. B. bei 475 nm) eine Transmission von nur etwa 70 % bis 75 %.

Aufgabe der Erfindung ist es daher, eine ophthalmologische Zusammensetzung der eingangs genannten Art zu schaffen, welche im wesentlichen den ganzen violetten Lichtanteil des sichtbaren Spektrums absorbiert und blaues Licht, insbesondere im Bereich zwischen 450 nm und 500 nm, möglichst wenig absorbiert.

Diese Aufgabe wird erfindungsgemäß durch die Merkmale des Patentanspruches 1 gelöst

Die ophthalmologische Zusammensetzung der Erfindung beinhaltet einen UV-Absorber, welcher Strahlung im Wellenlängenbereich von etwa 200 nm bis 400 nm absorbiert. Ferner beinhaltet die ophthalmologische Zusammensetzung einen Violett-Absorber, welcher violettes Licht im Wellenlängen von etwa 400 nm bis 430 nm absorbiert. Geeignete Farbstoffe des Violett-Absorbers sind Verbindungen, in denen eine Acrylat- oder Methacrylat-Einheit an einen unsubstituierten oder substituierten Pyrrolidinring gebunden ist.

Als UV-Absorber beinhaltet die ophthalmologische Zusammensetzung ein biokompaktibles UV-Lichtschutzmittel, wofür beispielsweise Benzotriazolderivate (Tinuvin®) verwendet werden können. Beispielsweise kann folgendes Lichtschutzmittel verwendet werden:

Methacrylsäure-(2-[3-(2H-benzotriazol-2-yl)4-hydroxy-phenyl]ethylester) mit der Struktur 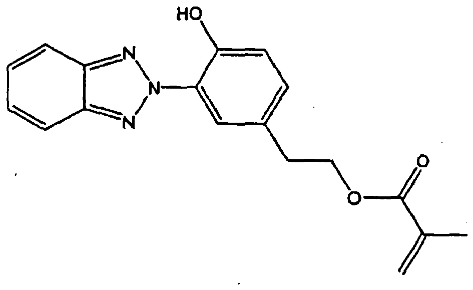 C₁₈H₁₇N₃O₃ / Molekulargewicht: 323,25 g/mol
CAS Nummer [96478-09-0]

Geeignete Violett-Absorber der erfindungsgemäßen ophthalinologischen Zusammensetzung sind Stereoisomere oder racemische Gemische von Verbindungen folgender Strukturen: 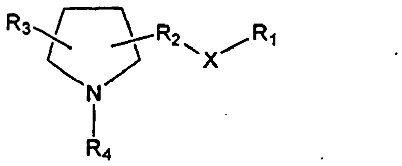 X = O, NH, NR5
R5 = Substituierter oder nicht substituierter Alkyl- oder Aryl-Rest (oder Kombination aus beiden) mit bis zu 30 Atomen, ausgewählt aus: C, H, Si, O, N, P, S, Cl Br, F;
R1 = Acryl- oder Methacrylrest 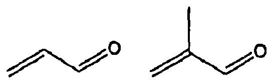 R2 = Organische Alkyl- oder Aryl-Spacergruppe (oder Kombination aus beiden) mit bis zu 30 Atomen, ausgewählt aus: C, H, Si, O, N, P, S, Cl, Br, F;
R3 = organische Substituentengruppe mit bis zu 30 Atomen, ausgewählt aus: C, H, Si, O, N, P, S, Cl, Br, F;
der Pyrrolidinring entweder nicht substituiert oder an einer Position (ein R3) bis mehrfach substituiert durch weitere Substituenten ist;
R4 = Organischer Alkyl- oder Aryl-Substituent (oder Kombination aus beiden) mit bis zu 30 Atomen, ausgewählt aus: C, H, Si, O, N, P, S, Cl, Br, F.

Beispiele entsprechender Strukturen (alle Stereoisomere oder racemische Gemische sind beinhaltet) sind:

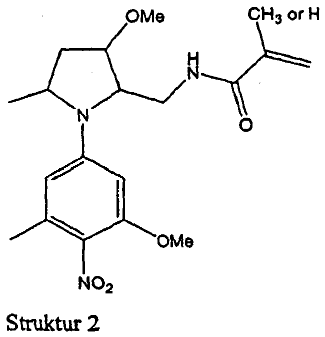

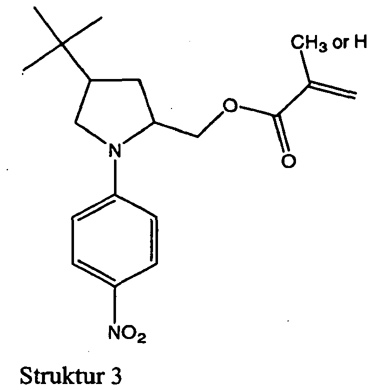

Ferner sind geeignete Violett-Absorber Stereoisomere oder racemischen Gemische von Verbindungen folgender Strukturen: 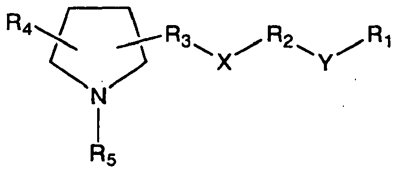 Y = O, NH, NR6;
X = O, NH, NR6;
R6 = Substituierter oder nicht substituierter Alkyl- oder Aryl-Rest (oder Kombination aus beiden) mit bis zu 30 Atomen, ausgewählt aus: C, H, Si, O, N, P, S, Cl, Br, F;
R1 = Acryl- oder Methacrylrest 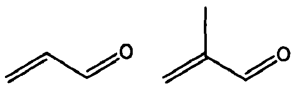 R2 = Organische Alkyl- oder Aryl-Spacergruppe (oder Kombination aus beiden) mit bis zu 30 Atomen, ausgewählt aus: C, H, Si, O, N, P, S, Cl, Br, F;
R3 = Organische Alkyl- oder Aryl-Spacergruppe (oder Kombination aus beiden) mit bis zu 30 Atomen, ausgewählt aus: C, H, Si, O, N, P, S, Cl, Br, F;
R4 = Organische Substituentengruppe mit bis zu 30 Atomen, ausgewählt aus: C, H, Si, O, N, P, S, Cl, Br, F;
der Pyrrolidinring entweder nicht substituiert oder an einer Position (ein R3) bis mehrfach substituiert durch weitere Substituenten ist;
R5 = Organischer Alkyl- oder Aryl-Substituent (oder Kombination aus beiden) mit bis zu 30 Atomen, ausgewählt aus: C, H, Si, O, N, P, S, Cl, Br, F;

Beispiele entsprechender Strukturen (alle Stereoisomere oder racemische Gemische sind beinhaltet) sind:

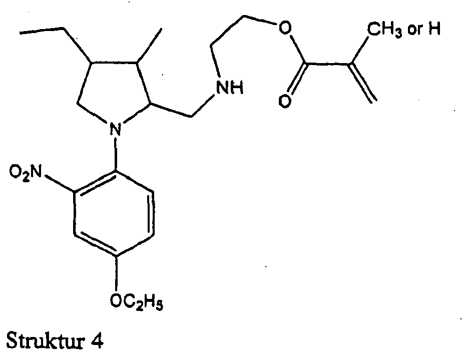

Ein bevorzugter Farbstoff für den Violett-Absorber der erfindungsgemäßen opthalmologischen Zusammensetzung ist ein

Acrylsäure(-(S)-1-(4-nitrophenyl)pyrrolidin-2-yl-methylester
mit der Struktur: 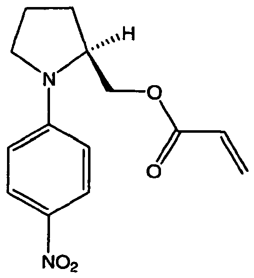 C₁₄H₁₆N₂O₄ / Molekulargewicht: 276,29 g/mol
CAS Nummer [152100-45-3]

Ausgehend von am Markt erhältlichen
(S)-(-)-1-(4-Nitrophenyl)-2-pyrrolidinemethanol
kann dieser Farbstoff mit folgenden Reaktionsschritten synthetisiert werden.

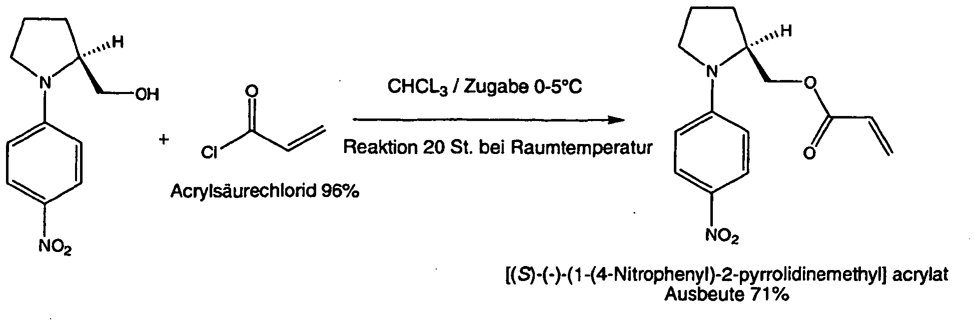

Alle möglichen Stereoisomere sind mitumfasst.

Ein bevorzugter Farbstoff hat folgende Struktur. Es handelt sich hierbei um einen Violett-Filter, der einen Methacryl-Rest beinhaltet

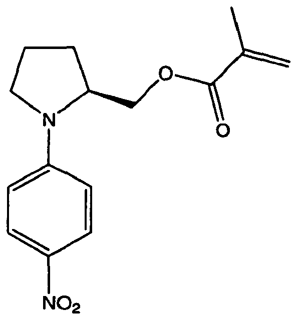

Die Herstellung dieser Verbindung erfolgt in zwei Schritten, wobei das Nitrophenylprolinol kommerziell erhältlich ist.

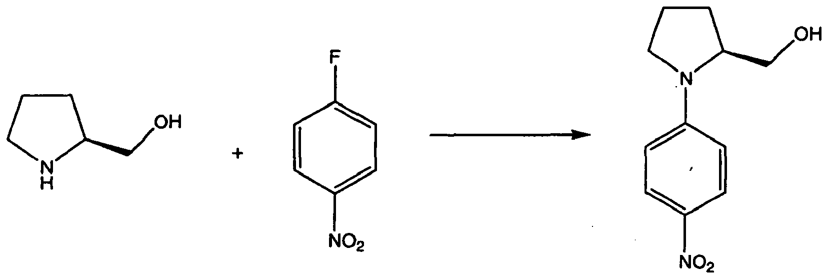

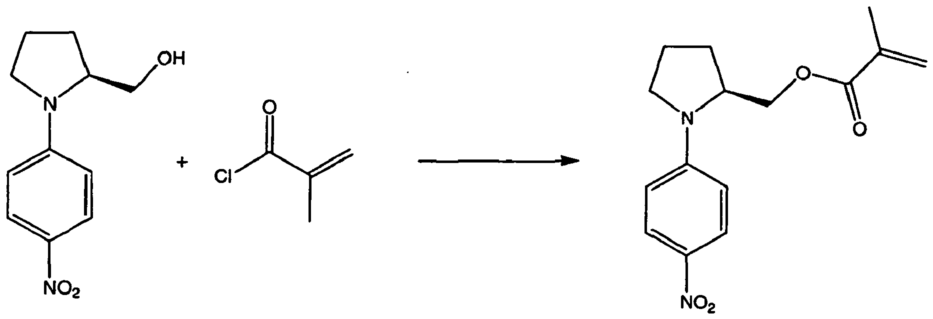

Ein weiteres Ausführungsbeispiel ist ein gelber Chromophor/Violett-Filter, dessen Molekül ein Nitrophenyl-Pyrrolidinemethylderivat ist. Die kovalente Anbindung der Verbindung zu dem Acryl- oder Methacryl-Rest hat keinen Einfluss auf das UV-Vis-Verhalten des Violett-Filters, da dieser Rest vom Chromophor durch eine CH₂-Einheit getrennt ist. Der Acryl- oder Methacryl-Rest dient zur kovalenten Einbindung des Violett-Filters in ein Trägermaterial, insbesondere Linsenmaterial auf Acrylat-Basis.

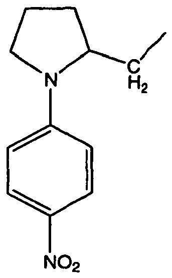

Weitere Ausführungsbeispiele für den Violett-Absorber sind Verbindungen, in denen ein Acryl- oder Methacryl-Rest an einen Nitrophenyl-pyrrolidin-Rest gebunden ist, wobei alle Stereoisomere mitumfasst sind, mit der Struktur: 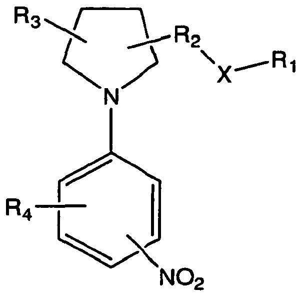 wobei
- R1 ist ein Acryl- bzw. Methacryl-Rest
- R2: organische verzweigte und unverzweigte Alkyl- oder Aryl-Substituenten (oder Kombination von beiden) mit bis zu 30 Atomen ausgewählt aus C, H, Si, O, N, P, S, F, Cl, Br;
- R3 und R4: H, organische verzweigte und unverzweigte Alkyl- oder Aryl- (oder Kombination von beiden) oder anderer Substituenten (zum Beispiel NO₂, OCH₃, OCH₂CG₃...) mit bis zu 30 Atomen ausgewählt aus C, H, Si, O, N, P, S, F, Cl, Br;
- X = O, S, NH, NR (R ist ein verzweigter und unverzweigter organischer Alkyl- oder Aryl-Substituent (oder Kombination von beiden) mit bis zu 30 Atomen ausgewählt aus C, H, Si, O, N, P, S, Cl, Br);

### Beispiel 1

### R2 = CH2, X = NR, R1 = Acryl- oder Methacrylrest, R3 und R4 = H.

Bei diesem Beispiel wird Nitrophenyl-Prolinol, welches kommerziell erhältlich ist, durch eine einfache synthetische Methode in das Amino-Derivat umgesetzt. Dieses Amin kann durch eine Reaktion mit Acrylsäurechlorid in das Amid umgewandelt. Die Struktur des Chromophors bleibt unverändert und ist durch die CH₂- Gruppe vom Acryl-Amid getrennt.

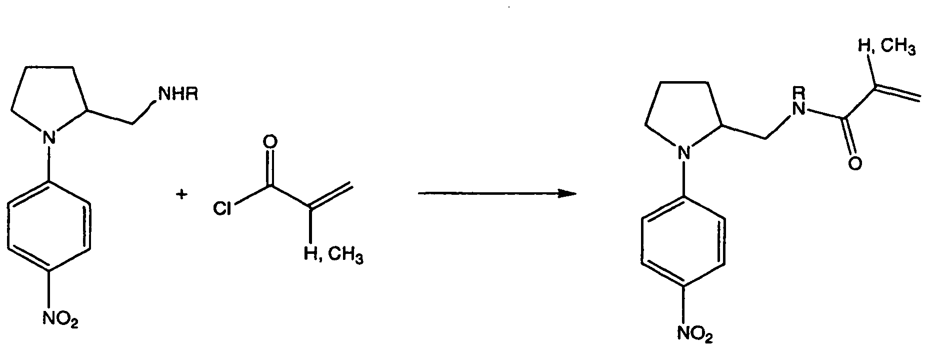

### Beispiel 2:

### R2 = CH2-O-CH(OH)CH2CH2, X = O, R1 = Methacrylrest, R3 und R4 = H

Setzt man Prolinol nicht mit Acrylsäurechlorid um, sondern mit kommerziell erhältlichem Methacrylsäure-glycidylester, so bekommt man in einem einzigen Schritt eine Verbindung mit dem Chromophor, der durch eine aliphatische Kette vom Methacrylat-Rest getrennt ist. Deshalb ist die Verbindung ein Violett-Filter.

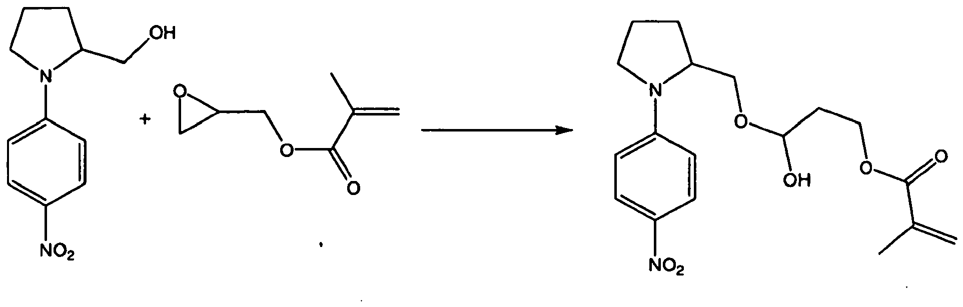

### Beispiel 3

Hier ist R4 eine Methylgruppe, die einen schwachen induktiven und einen schwachen mesomeren Effekt besitzt. Die entstehende Verbindung ist ein Violett-Filter, R3 = H. Das Einbringen einer zusätzlichen CH₃-Gruppe in den oben beschriebenen bevorzugten Violett-Filter ist synthetisch ohne Probleme zu bewältigen. Setzt man das Prolinol nicht mit 1-Fluor-4-Nitrophenyl, sondern mit 2-Fluor-5-Nitrotuluol (kommerziell erhältlich) um, so wird ein Prolinol erzeugt, in dem R4 eine CH₃-Gruppe ist. Durch einfache Veresterung mit Acrylsäurechlorid oder Methacrylsäurechlorid erhält man eine Verbindung, die sich vom oben beschriebenen Filter durch eine zusätzliche Methylgruppe unterscheidet.

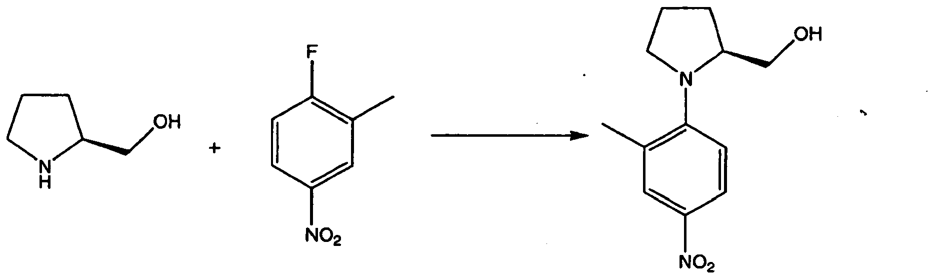

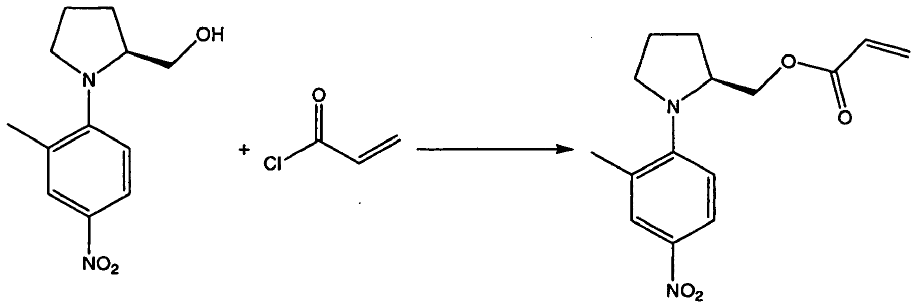

### Beispiel 4

Hier ist R3 eine CH₃-Gruppe und R4 = H. Das Chromophor ist ein Violett-Filter. Setzt man das durch Reaktion hergestellte Methyl-Prolinol durch die unten gezeigte Synthese um, so wird ein Prolinol erzeugt, in dem R3 eine CH₃-Gruppe ist. Der dann durch Synthese hergestellte Acrylat-Filter unterscheidet sich von dem bevorzugten Filter nur durch eine zusätzliche Methylgruppe.

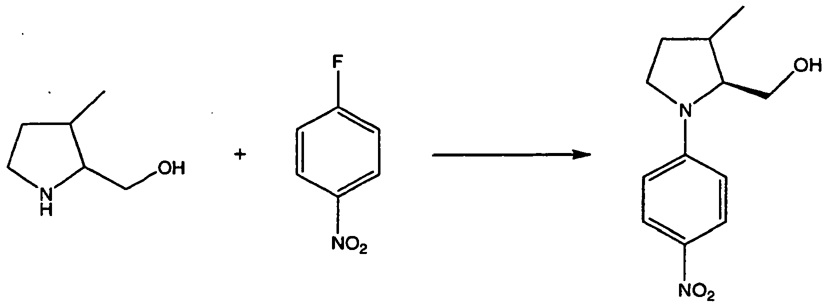

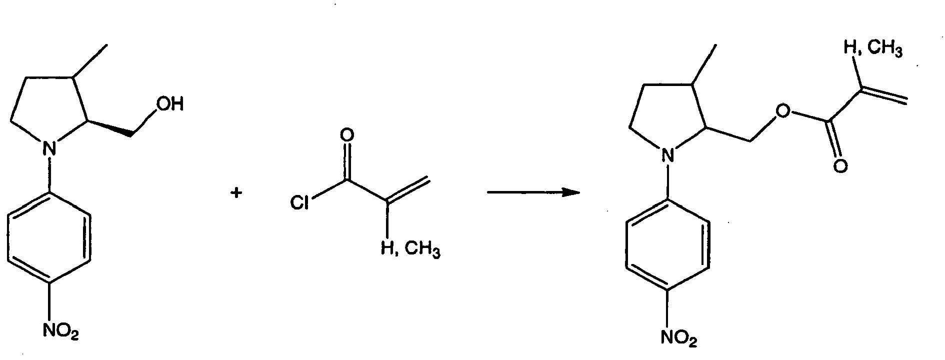

Als Trägermaterial, insbesondere biokompatibles Trägermaterial sind Acrylate, insbesondere mit einem Wassergehalt von 1 % bis 28 %, für die ophthalmologische Zusammensetzung geeignet. In diesem Trägermaterial sind der UV-Absorber und der Violett-Absorber vorhanden und insbesondere kovalent gebunden. Der UV-Absorber ist vorzugsweise in einem Konzentrationsbereich von 0,5 % bis 1,0 % vorhanden. Wenn die ophthalmologische Zusammensetzung für eine Intraokularlinse verwendet wird, hängt die jeweilige Konzentration des UV-Absorbers vom jeweiligen Scheitelbrechwert (Dioptrie) der Linse ab. Der Violett-Absorber ist ebenfalls vorzugsweise kovalent im Acrylat-Trägermaterial gebunden. Die Gefahr des Auswaschens aus der Trägermatrix besteht nicht. Er kann in einem Konzentrationsbereich von 0,02 % bis 0,16 % vorliegen. Bei Verwendung der ophthalmologischen Zusammensetzung für eine Intraokularlinse hängt die Konzentration des Violett-Absorbers ebenfalls vom Scheitelbrechwert (Dioptrie) der Linse ab.

Geeignete biokompatible Trägermaterialien für die ophthalmologische Zusammensetzung sind beispielsweise HEMA (Hydroxyethylmethacrylat) und MMA (Methylmethacrylat). Auch Copolymerisate aus diesen Stoffen unter Verwendung von vorzugsweise EGDMA (Ethylenglykoldimethacrylat) als Quervemetzer können als Acrylat-Trägermaterial verwendet werden. Die Trägermaterialien können hydrophil mit einem Wassergehalt von beispielsweise 1 % bis 30% oder hydrophob ausgebildet sein.

Ausführungsbeispiele der ophthalmologischen Zusammensetzung sind folgende mit quantitativen Zusammensetzungen in Gewichts%:

### Beispiel 1

| | |
|---|---|
| EA (Ethylacrylat): | >45-<75% |
| EMA (Ethylmethacrylat): | 25-50% |
| TFEMA (Trifluorethylmethacrylat): | 3-10% |
| EGDMA (Ethylenglykoldimethacrylat): | 0,25-1,5% |
| AIBN (α, α'-Azoisobutyronitril): | 0,01-0,1% |
| UV-Absorber: | 0,1-1,0% |
| Violett-Absorber: | 0,03-0,16% |

Der Glaspunkt dieses hydrophoben, wasserfreien Polymers liegt bevorzugt im Bereich 0-11°C

### Beispiel 2

| | |
|---|---|
| HEMA (Hydroxyethylmethacrylat): | >80-<90% |
| MMA (Methylmethacrylat): | 10-16% |
| EGDMA (Ethylenglykoldimethacrylat): | 0,25-1,5% |
| AIBN (α, α'-Azoisobutyronitril): | 0,01-0,1 % |
| UV-Absorber: | 0,1-1,0% |
| Violett-Absorber: | 0,03-0,16% |

Der Wassergehalt des hydratisierten Polymers liegt bevorzugt bei 26-30%

Zur Synthese der jeweiligen Linsenmaterialien werden zur Herstellung des hydrophilen Materials in ein Becherglas nacheinander AIBN, Violett-Absorber, UV-Absorber, EGDMA sowie MMA eingewogen und solange gerührt, bis alles gelöst ist. Anschließend wird HEMA zugegeben und nochmals für ca. 10 Minuten gerührt.

Bei der Herstellung des hydrophoben Materials werden nacheinander AIBN, Violett-Absorber, UV-Absorber, EGDMA, TFEMA, EA sowie EMA in ein Becherglas eingewogen und auf dem Magnetrührer so lange gerührt bis sich alles gelöst hat.

Die sich jeweils ergebende Mischung wird mittels eines geeigneten Filtersystems abfiltriert und in die Polamerisationsformen überführt (z.B. Näpfe, Stangen oder Plattformen). Die Polymerisaton wird durch Erhitzen eingeleitet (z.B. 40-60°C für 4-24h sowie 70-130°C für weitere 4-24h). Nach dem Erkalten werden die Polymerisate entnommen und durch Drehen und Fräsen auf die gewünschte Rohlingsgröße gebracht (z.B. 3mm Stärke, 12,7mm Durchmesser).

Die Ausführungsbeispiele haben folgende Transmissionseigenschaften:

| Wellenlänge (nm) | Transmission % (20, 0 Dpt) |
|---|---|
| 390 | < 2% |
| 400 | < 2% |
| 420 | < 2% |
| 430 | 5 % |
| 450 | 31 % |
| 475 | 82 % |
| 490 | 92 % |
| 500 | 93% |
| 525 | 93% |
| 550 | 93% |
| 600 | 93% |
| 700 | 93% |

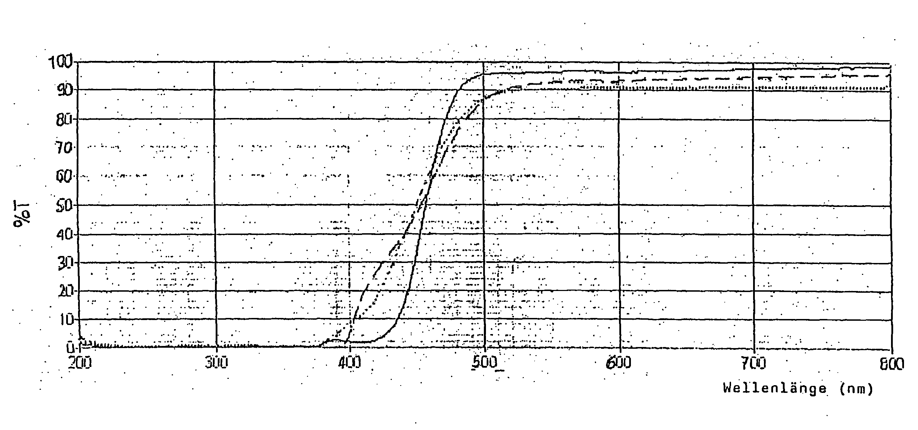

In der beigefügten Figur ist das Transmissionsverhalten der erfindungsgemäßen ophthalmologischen Zusammensetzung verglichen mit den Transmissionsverhalten zweier bekannter Zusammensetzungen für Intraokularlinsen. Auf der Abzisse sind die Wellenlängen in nm und auf der Ordinate das Transmissionsverhalten in Prozent angegeben. Das Transmissionsverhalten der erfindungsgemäßen Zusammensetzung wird mit einer durchgezogenen Linie und die Transmissionsverhalten der bekannten Intraokularlinsen werden punktiert und strichliert dargestellt. Hieraus ist ersichtlich, dass mit Hilfe der erfindungsgemäßen ophthalmologischen Zusammensetzung nicht nur der UV-Anteil (< 400 nm), sondern auch der gesamte violette Lichtanteil (400 nm bis 430 nm) absorbiert wird. Die bekannten Zusammensetzungen haben im violetten Bereich eine hohe Lichtdurchlässigkeit mit bis zu einem Drittel des violetten Anteils. Die erfindungsgemäße Zusammensetzung hat bei 430 nm lediglich eine Transmission von 5 %.

Im blauen Lichtbereich hat die erfindungsgemäße Zusammensetzung beispielsweise bei 475 nm eine Lichtdurchlässigkeit von 82 %, während die bekannten Linsen hier nur 70 % bzw. 75 % haben.

Die ophthalmologische Zusammensetzung eignet sich insbesondere für Sehhilfen, wie Brillen, Kontaktlinsen, Augenimplantate, wie Homhautimplantante und insbesondere eignet sich die erfindungsgemäße ophthalmologische Zusammensetzung für Intraokularlinsen.

## Patentansprüche

1. Ophthalmologische Zusammensetzung, welche ausschließlich auf Acrylat- und/oder Methacrylatbasis aufgebaut ist und welche einen UV-Absorber und ferner einen Farbstoff, bei dem eine Acrylsäure- oder Methacrylsäure-Einheit an einem substituierten oder unsubstituierten Pyrrolidinring gebunden ist, als Violett-Absorber aufweist.

2. Ophthalmologische Zusammensetzung nach Anspruch 1, bei welcher der Violett-Absorber ein Stereoisomer oder racemisches Gemisch von Farbstoffen folgender Struktur ist: 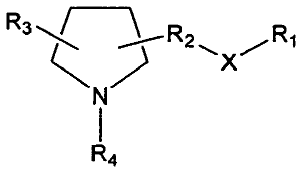 wobei
R1 = ein Acryl- oder Methacrylrest
R2 = Organische Alkyl- oder Aryl-Spacergruppe (oder Kombination aus beiden) mit bis zu 30 Atomen, ausgewählt aus: C, H, Si, O, N, P, S, Cl, Br, F;
R3 = Organische Substituentengruppe mit bis zu 30 Atomen, ausgewählt aus: C, H, Si, O, N, P, S, Cl, Br, F;
der Pyrrolidinring entweder nicht substituiert oder an einer Position (ein R3) bis mehrfach substituiert durch weitere Substituenten ist;
R4 = Organischer Alkyl- oder Aryl-Substituent (oder Kombination aus beiden) mit bis zu 30 Atomen, ausgewählt aus: C, H, Si, O, N, P, S, Cl, Br, F;
X = O, NH; NR5;
R5 = Substituierter oder nicht substituierter Alkyl- oder Aryl-Rest (oder Kombination aus beiden) mit bis zu 30 Atomen, ausgewählt aus: C, H, Si, O, N, P, S, Cl, Br, F;

3. Ophthalmologische Zusammensetzung nach Anspruch 1, bei welcher derViolett-Absorber ein Stereoisomer oder racemisches Gemisch von Farbstoffen folgender Struktur ist: 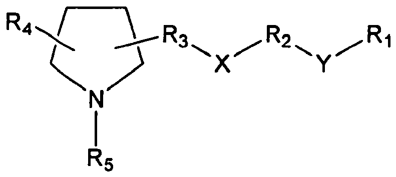 wobei
Y = O, NH, NR6;
X = O, NH, NR6;
R6 = Substituierter oder nicht substituierter Alkyl- oder Aryl-Rest (oder Kombination aus beiden) mit bis zu 30 Atomen, ausgewählt aus: C, H, Si, O, N, P, S, Cl, Br, F; R1 = ein Acryl- oder Methacrylrest
R2 = Organische Alkyl- oder Aryl-Spacergruppe (oder Kombination aus beiden) mit bis zu 30 Atomen, ausgewählt aus: C, H, Si, O, N, P, S, Cl, Br, F;
R3 = Organische Alkyl- oder Aryl-Spacergruppe (oder Kombination aus beiden) mit bis zu 30 Atomen, ausgewählt aus: C, H, Si, O, N, P, S, Cl, Br, F;
R4 = Organische Substituentengruppe mit bis zu 30 Atomen, ausgewählt aus: C, H, Si, O, N, P, S, Cl, Br, F;
der Pyrrolidinring entweder nicht substituiert oder an einer Position (ein R4) bis mehrfach substituiert durch weitere Substituenten ist;
R5 = Organischer Alkyl- oder Aryl-Substituent (oder Kombination aus beiden) mit bis zu 30 Atomen, ausgewählt aus: C, H, Si, O, N, P, S, Cl, Br, F.

4. Ophthalmologische Zusammensetzung nach Anspruch 1, **dadurch gekennzeichnet, dass** der Violett-Absorber ein Farbstoff folgender Struktur ist: 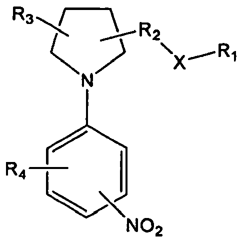 wobei
• R1 ist ein Acryl- oder Methacryl-Rest
• R2: organische verzweigte und unverzweigte Alkyl- oder Aryl-Substituenten (oder Kombination von beiden) mit bis zu 30 Atomen ausgewählt aus C, H, Si, O, N, P, S, F, Cl, Br;
• R3 und R4: H, organische verzweigte und unverzweigte Alkyl- oder Aryl-(oder Kombination von beiden) oder anderer Substituenten (zum Beispiel NO₂, OCH₃, OCH₂CH₃...) mit bis zu 30 Atomen ausgewählt aus C, H, Si, O, N, P, S, F, Cl, Br; und
• X = O, S, NH, NR (R ist ein verzweigter oder unverzweigter organischer Alkyl- oder Aryl-Substituent (oder Kombination von beiden) mit bis zu 30 Atomen ausgewählt aus C, H, Si, O, N, P, S, Cl, Br.

5. Ophthalmologische Zusammensetzung nach Anspruch 4, **dadurch gekennzeichnet, dass** R2 = CH2, X = NR, R1 ist ein Acryl- oder Methacryl-Rest, R3 und R4 = H mit der Struktur 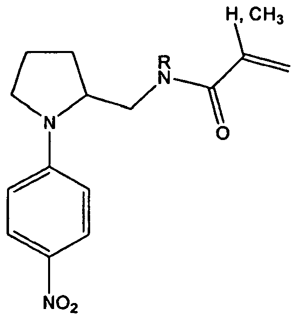 ist.

6. Ophthalmologische Zusammensetzung nach Anspruch 4, **dadurch gekennzeichnet, dass** R2 = CH₂-O-CH(OH)CH₂CH₂, X = O, R3 und R4 = H und R1 = Methacrylrest mit der Struktur 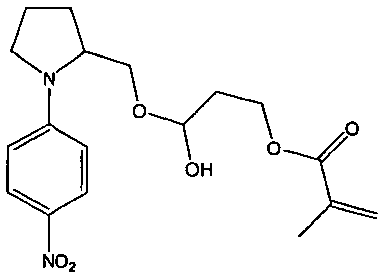 ist.

7. Ophthalmologische Zusammensetzung nach Anspruch 4, **dadurch gekennzeichnet, dass** R4 = CH₃, R1 = Acryl-Rest, X = O, R2 = CH₂, R3 = H mit der Struktur 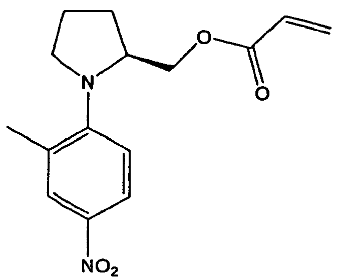 ist.

8. Ophthalmologische Zusammensetzung nach Anspruch 4, **dadurch gekennzeichnet, dass** R3 eine Methylgruppe, R1 = Acryl- oder Methacryl-Rest, X = O, R2 = CH₂, R4 = H mit der Struktur 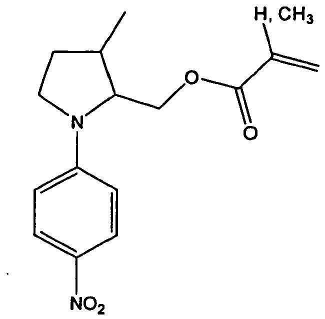 ist.

9. Ophthalmologische Zusammensetzung nach Anspruch 1, bei welcher der Violett-Absorber ein
Methacrylsäure(-(S)-1-(4-nitrophenyl)pyrrolidin-2-yl-methylester mit der Struktur: 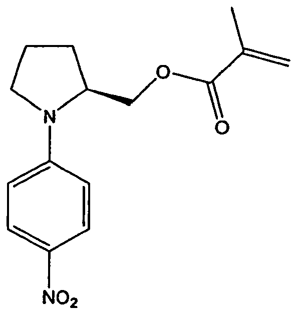 ist.

10. Ophthalmologische Zusammensetzung nach einem der Ansprüche 1 bis 9, **dadurch gekennzeichnet, dass** der UV-Absorber Methacrylsäure-(2-[3-(2H-benzotriazol-2-yl)4-hydroxy-phenyl]ethylester) mit der Struktur 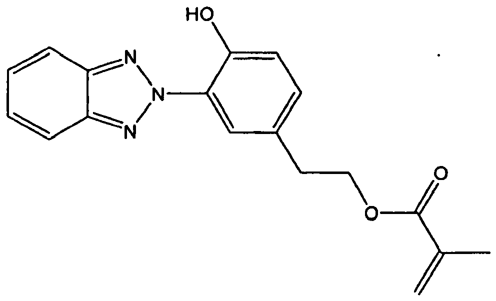 ist.

11. Ophthalmologische Zusammensetzung nach einem der Ansprüche 1 bis 10, enthaltend
| | |
|---|---|
| HEMA (Hydroxyethylmethacrylat): | >80-<90% |
| MMA (Methylmethacrylat): | 10-16% |
| EGDMA (Ethylenglykoldimethacrylat): | 0,25-1,5% |
| AIBN (α, α'-Azoisobuthyronitril): | 0,01-0,1% |
| UV-Absorber: | 0,1-1,0% |
| Violett-Absorber: | 0,02-0,16% |

12. Ophthalmologische Zusammensetzung nach einem der Ansprüche 1 bis 10, enthaltend:
| | |
|---|---|
| EA (Ethylacrylat): | >45-<75% |
| EMA (Ethylmethylacrylat): | 25-50% |
| TFEMA (Trifluorethylmethacrylat): | 3-10% |
| EGDMA (Ethylenglykoldimethacrylat): | 0,25-1,5% |
| AIBN (α, α'-Azoisobuthyronitril): | 0,01-0,1% |
| UV-Absorber: | 0,1-1,0% |
| Violett-Absorber: | 0,03-0,16% |

13. Verwendung einer ophthalmologischen Zusammensetzung nach einem der Ansprüche 1 bis 12 zur Herstellung einer ophthalmischen Linse, insbesondere eines ophthalmologischen Implantats, insbesondere einer Intraokularlinse.

14. Augenimplantat, dessen Implantatmaterial eine ophthalmologische Zusammensetzung nach einem der Ansprüche 1 bis 12 aufweist.

15. Augenimplantat nach Anspruch 14, welches als Intraokularlinse ausgebildet ist.

## Claims

1. An ophthalmologic composition constructed exclusively based on acrylate and/or methacrylate and having an UV absorber and further a dye, in which an acrylic acid or methacrylic acid unit is bound to a substituted or unsubstituted pyrrolidine ring, as a violet absorber.

2. The ophthalmologic composition according to claim 1, in which the violet absorber is a stereoisomer or racemic mixture of dyes of the following structure: 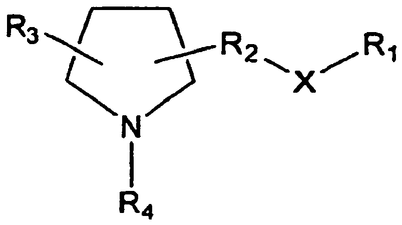 wherein
R1 = an acryl or methacryl radical
R2 = organic alkyl or aryl spacer group (or combination of both) with up to 30 atoms, selected from: C, H, Si, O, N, P, S, Cl, Br, F;
R3 = organic substituent group with up to 30 atoms, selected from: C, H, Si, O, N, P, S, Cl, Br, F;
the pyrrolidine ring is either not substituted or substituted in a position (an R3) up to several times by further substituents;
R4 = organic alkyl or aryl substituent (or combination of both) with up to 30 atoms, selected from: C, H, Si, O, N, P, S, Cl, Br, F;
X = O, NH; NR5;
R5 = substituted or unsubstituted alkyl or aryl radical (or combination of both) with up to 30 atoms, selected from: C, H, Si, O, N, P, S, Cl, Br, F;

3. The ophthalmologic composition according to claim 1, in which the violet absorber is a stereoisomer or racemic mixture of dyes of the following structure: 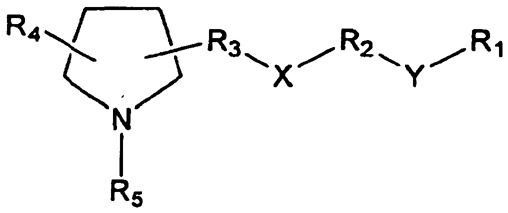 wherein
Y = O, NH, NR6;
X = O, NH, NR6;
R6 = substituted or unsubstituted alkyl or aryl radical (or combination of both) with up to 30 atoms, selected from: C, H, Si, O, N, P, S, Cl, Br, F;
R1 = an acryl or methacryl radical
R2 = organic alkyl or aryl spacer group (or combination of both) with up to 30 atoms, selected from: C, H, Si, O, N, P, S, Cl, Br, F;
R3 = organic alkyl or aryl spacer group (or combination of both) with up to 30 atoms, selected from: C, H, Si, O, N, P, S, Cl, Br, F;
R4 = organic substituent group with up to 30 atoms, selected from: C, H, Si, O, N, P, S, Cl, Br, F;
the pyrrolidine ring is either not substituted or substituted in a position (an R4) up to several times by further substituents;
R5 = organic alkyl or aryl substituent (or combination of both) with up to 30 atoms, selected from: C, H, Si, O, N, P, S, Cl, Br, F.

4. The ophthalmologic composition according to claim 1, **characterized in that** the violet absorber is a dye of the following structure: 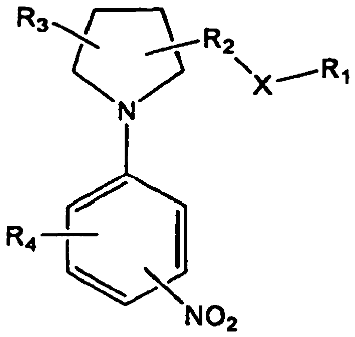 wherein
• R1 is an acryl or methacryl radical
• R2: organic branched and unbranched alkyl or aryl substituents (or combination of both) with up to 30 atoms, selected from C, H, Si, O, N, P, S, F, Cl, Br;
• R3 and R4: H, organic branched and unbranched alkyl or aryl (or combination of both) or other substituents (for example NO₂, OCH₃, OCH₂CH₃...) with up to 30 atoms, selected from C, H, Si, O, N, P, S, F, Cl, Br; and
• X = O, S, NH, NR (R is a branched or unbranched organic alkyl or aryl substituent (or combination of both) with up to 30 atoms, selected from C, H, Si, O, N, P, S, Cl, Br).

5. The ophthalmologic composition according to claim 4, **characterized in that** R2 = CH2, X = NR, R1 is an acryl or methacryl radical, R3 and R4 = H with the structure

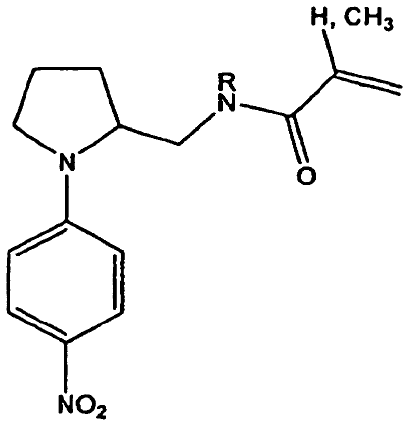

6. The ophthalmologic composition according to claim 4, **characterized in that** R2 = CH₂-O-CH(OH)CH₂CH₂, X = O, R3 and R4 = H and R1 = methacryl radical with the structure

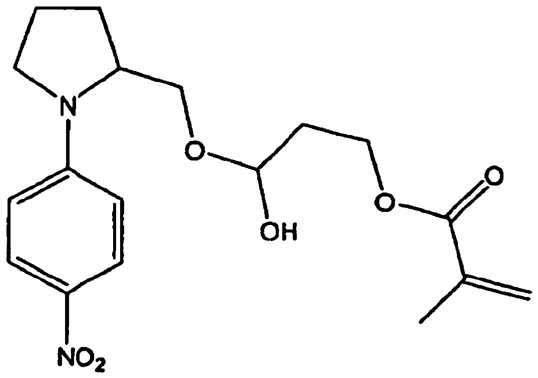

7. The ophthalmologic composition according to claim 4, **characterized in that** R4 = CH₃, R1 = acryl radical, X = O, R2 = CH₂, R3 = H with the structure

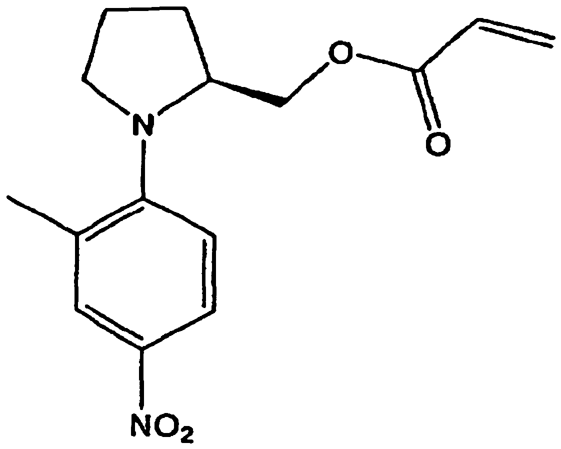

8. The ophthalmologic composition according to claim 4, **characterized in that** R3 is a methyl group, R1 = acryl or methacryl radical, X = O, R2 = CH₂, R4 = H with the structure

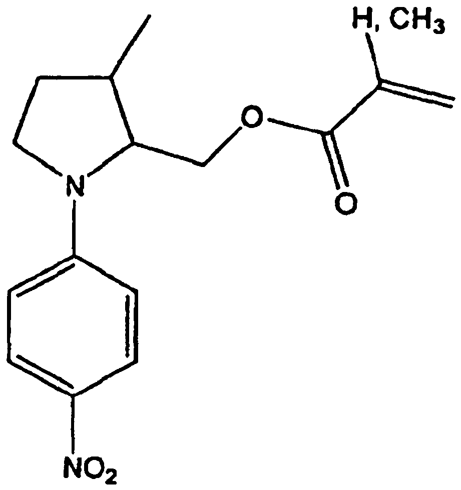

9. The ophthalmologic composition according to claim 1, in which the violet absorber is a methacrylic acid (-(S)-1-(4-nitrophenyl)pyrrolidine-2-yl-methyl ester with the structure:

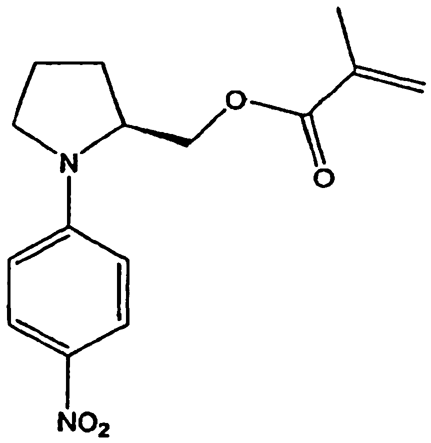

10. The ophthalmologic composition according to any one of claims 1 to 9, **characterized in that** the UV absorber is methacrylic acid (2-[3-(2H-benzotriazol-2-yl)4-hydroxy-phenyl]ethyl ester) with the structure

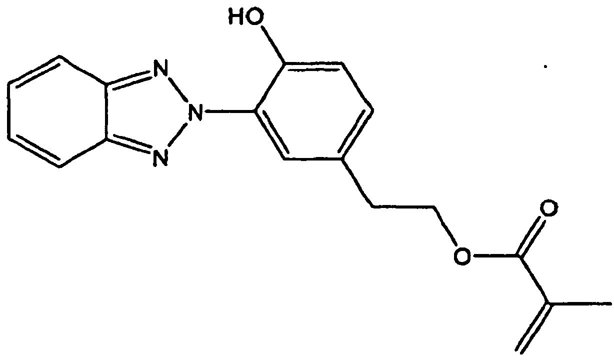

11. The ophthalmologic composition according to any one of claims 1 to 10, containing
| | |
|---|---|
| HEMA (hydroxyethyl methacrylate): | >80-<90 % |
| MMA (methyl methacrylate): | 10-16 % |
| EGDMA (ethylene glycol dimethacrylate): | 0.25-1.5 % |
| AIBN (α,α'-azoisobutyronitrile): | 0.01-0.1 % |
| UV absorber: | 0.1-1.0 % |
| violet absorber: | 0.02-0.16 % |

12. The ophthalmologic composition according to any one of claims 1 to 10, containing:
| | |
|---|---|
| EA (ethyl acrylate): | >45-<75 % |
| EMA (ethyl methacrylate): | 25-50 % |
| TFEMA (trifluoroethyl methacrylate): | 3-10 % |
| EGDMA (ethylene glycol dimethacrylate): | 0.25-1.5 % |
| AIBN (α,α'-azoisobutyronitrile): | 0.01-0.1 % |
| UV absorber: | 0.1-1.0% |
| violet absorber: | 0.03-0.16% |

13. A use of an ophthalmologic composition according to any one of claims 1 to 12 for producing an ophthalmic lens, in particular of an ophthalmologic implant, in particular of an intraocular lens.

14. An eye implant, the implant material of which has an ophthalmologic composition according to any one of claims 1 to 12.

15. The eye implant according to claim 14, which is formed as an intraocular lens.

## Revendications

1. Composition ophtalmologique laquelle est uniquement constituée sur la base d'acrylate et/ou de méthacrylate et laquelle présente un absorbant ultraviolet et en outre un colorant, dans lequel une unité d'acide acrylique ou d'acide méthacrylique est liée à un anneau de pyrrolidine substitué ou non substitué, en tant qu'absorbant violet.

2. Composition ophtalmologique selon la revendication 1, dans laquelle l'absorbant violet est un stéréo-isomère ou un mélange racémique de colorants de la structure suivante : 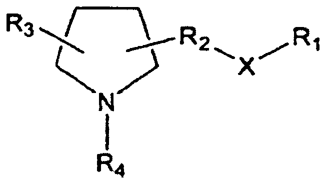 où
R1 = un radical acrylique ou méthacrylique
R2 = groupe espaceur alkyle ou aryle organique (ou combinaison des deux) comprenant jusqu'à 30 atomes, sélectionné à partir de : C, H, Si, O, N, P, S, CI, Br, F ;
R3 = groupe organique de substituants comprenant jusqu'à 30 atomes, sélectionné à partir de : C, H, Si, O, N, P, S, Cl, Br, F ;
l'anneau de pyrrolidine est soit non substitué soit substitué jusqu'à plusieurs fois à une position (un R3) par d'autres substituants ;
R4 = substituant alkyle ou aryle organique (ou combinaison des deux) comprenant jusqu'à 30 atomes, sélectionné à partir de : C, H, Si, O, N, P, S, Cl, Br, F ; X = O, NH ; NR5 ;
R5 = radical alkyle ou aryle substitué ou non substitué (ou combinaison des deux) comprenant jusqu'à 30 atomes, sélectionné à partir de : C, H, Si, O, N, P, S, Cl, Br. F ;

3. Composition ophtalmologique selon la revendication 1, dans laquelle l'absorbant violet est un stéréo-isomère ou un mélange racémique de colorants de la structure suivante : 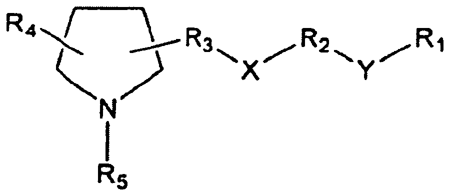 où
Y = O, NH, NR6 ;
X = O, NH, NR6 ;
R6 = radical alkyle ou aryle substitué ou non substitué (ou combinaison des deux) comprenant jusqu'à 30 atomes, sélectionné à partir de : C, H, Si, O, N, P, S, Cl, Br, F ; R1 = un radical acrylique ou méthacrylique
R2 = groupe espaceur alkyle ou aryle organique (ou combinaison des deux) comprenant jusqu'à 30 atomes, sélectionné à partir de : C, H, Si, O, N, P, S, Cl, Br, F ;
R3 = groupe espaceur alkyle ou aryle organique (ou combinaison des deux) comprenant jusqu'à 30 atomes, sélectionné à partir de : C, H, Si, O, N, P, S, Cl, Br, F ;
R4 = groupe organique de substituants comprenant jusqu'à 30 atomes, sélectionné à partir de : C, H, Si, O, N, P, S, Cl, Br, F ;
l'anneau de pyrrolidine est soit non substitué soit substitué jusqu'à plusieurs fois à une position (un R4) par d'autres substituants ;
R5 = substituant alkyle ou aryle organique (ou combinaison des deux) comprenant jusqu'à 30 atomes, sélectionné à partir de : C, H, Si, O, N, P, S, Cl, Br, F.

4. Composition ophtalmologique selon la revendication 1, dans laquelle l'absorbant violet est un colorant de la structure suivante : 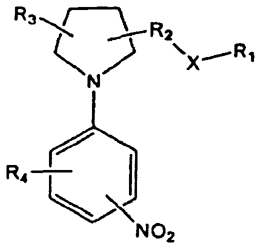 où
• R1 est un radical acrylique ou méthacrylique
• R2 : substituants alkyles ou aryles organiques ramifiés et non ramifiés (ou combinaison des deux) comprenant jusqu'à 30 atomes, sélectionnés à partir de C, H, Si, O, N, P, S, F, Cl, Br ;
• R3 et R4 : substituants alkyles ou aryles organiques ramifiés et non ramifiés (ou combinaison des deux) ou autres substituants (par exemple NO₂, OCH₃, OCH₂CH₃ ...) comprenant jusqu'à 30 atomes, sélectionnés à partir de C, H, Si, O, N, P, S, F, Cl, Br ; et
• X = O, S, NH, NR (R étant un substituant alkyle ou aryle organique ramifié ou non ramifié (ou combinaison des deux) comprenant jusqu'à 30 atomes, sélectionnés à partir de C, H, Si, O, N, P, S, Cl, Br.

5. Composition ophtalmologique selon la revendication 4, **caractérisée en ce que** R2 = CH2, X = NR, R1 étant un radical acrylique ou méthacrylique, R3 et R4 = H avec la structure

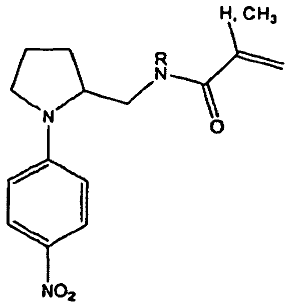

6. Composition ophtalmologique selon la revendication 4, **caractérisée en ce que** R2 = CH₂-O-CH(OH)CH₂CH₂, X = O, R3 et R4 = H et R1 = radical méthacrylique avec la structure

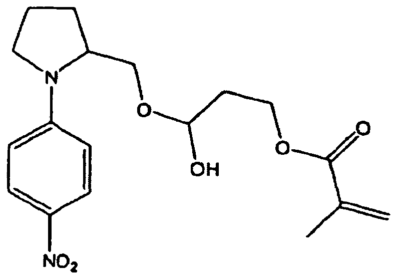

7. Composition ophtalmologique selon la revendication 4, **caractérisée en ce que** R4 = CH₃, R1 = radical acrylique, X = O, R2 = CH₂, R3 = H avec la structure

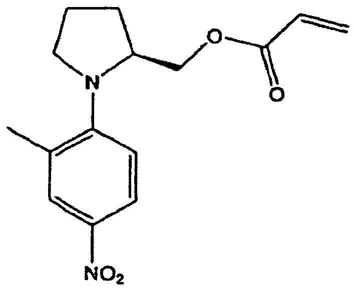

8. Composition ophtalmologique selon la revendication 4, **caractérisée en ce que** R3 est un groupe méthyle, R1 = radical acrylique ou méthacrylique, X = O, R2 = CH₂, R4 = H avec la structure

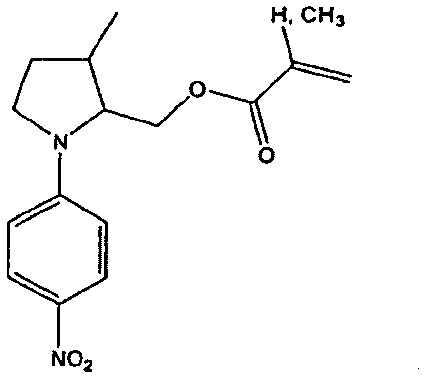

9. Composition ophtalmologique selon la revendication 1, dans laquelle l'absorbant violet est un
acide méthacrylique(-(S)-1-(4-nitrophényle)pérrolidine-2-yl-méthylester avec la structure

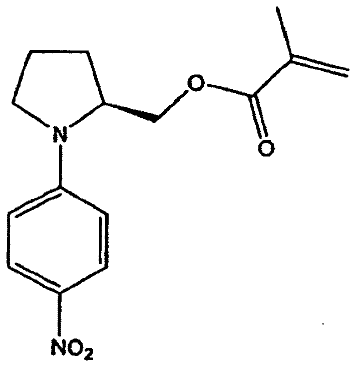

10. Composition ophtalmologique selon l'une quelconque des revendications 1 à 9,
**caractérisée en ce que** l'absorbant ultraviolet est un
acide méthacrylique-(2-[3-(2H-benzotriazol-2-yl)4-hydroxy-phényle]éthylester) avec la structure

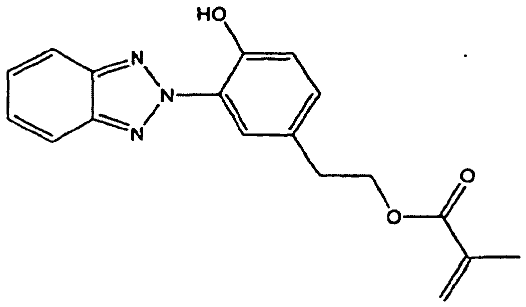

11. Composition ophtalmologique selon l'une quelconque des revendications 1 à 10, comprenant
| | |
|---|---|
| HEMA (méthacrylate d'hydroxyéthyle) : | >80-<90% |
| MMA (méthacrylate de méthyle) : | 10-16% |
| EGDMA (diméthacrylate d'alcool éthylique) : | 0,25-1,5% |
| AIBN (azoisobutyronitrile α, α') : | 0,01-0,1% |
| Absorbant ultraviolet : | 0,1-1,0% |
| Absorbant violet : | 0,02-0,16% |

12. Composition ophtalmologique selon l'une quelconque des revendications 1 à 10, contenant
| | |
|---|---|
| EA (acrylate d'éthyle) : | >45-<75% |
| EMA (éthylène-acrylate de méthyle) : | 25-50% |
| TFEMA (méthacrylate de trifluoroéthyle) : | 3-10% |
| EGDMA (diméthacrylate d'éthylène glycol) : | 0,25-1,5% |
| AIBN (azoisobutyronitrile α, α') : | 0,01-0,1% |
| Absorbant ultraviolet : | 0,1-1,0% |
| Absorbant violet : | 0,03-0,16% |

13. Utilisation d'une composition ophtalmologique selon l'une quelconque des revendications 1 à 12, destinée à la fabrication d'une lentille ophtalmologique, notamment d'un implant ophtalmologique, notamment d'une lentille intraoculaire.

14. Implant oculaire, dont le matériau d'implant présente une composition ophtalmologique selon l'une quelconque des revendications 1 à 12.

15. Implant oculaire selon la revendication 14, lequel est réalisé en tant que lentille intraoculaire.
